(19) Europäisches Patentamt
European Patent Office
Office européen des brevets

(11) Veröffentlichungsnummer: **0 241 864**
**A2**

(12) # EUROPÄISCHE PATENTANMELDUNG

(21) Anmeldenummer: **87105261.9**

(22) Anmeldetag: **09.04.87**

(51) Int. Cl.4: **C07K 5/06**, C12P 21/02, //C07K1/14

(30) Priorität: **12.04.86 DE 3612344**

(43) Veröffentlichungstag der Anmeldung:
**21.10.87 Patentblatt 87/43**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmidt, Erwin**
**Am Flachsland 26**
**D-6233 Kelkheim Taunus(DE)**
Erfinder: **Keller, Reinhold**
**Wiesenweg 5**
**D-6232 Bad Soden am Taunus(DE)**
Erfinder: **Schlingmann, Merten**
**Schneidhainer Strasse 32a**
**D-6240 Königstein Taunus(DE)**

(54) **Verfahren zur Herstellung von Dipeptid-Additionsverbindungen und den daraus freigesetzten Dipeptiden.**

(57) Bei der enzymatischen Verknüpfung von D-,L-Phenylalaninalkylester oder L-Phenylalaninalkylester mit einer N-geschützten L-Asparaginsäure können eine Phenoxyacetyl-, Phenoxypropionyl-oder Phenylacetylgruppe als Aminoschutzgruppe verwendet werden.



Xerox Copy Centre

# Verfahren zur Herstellung von Dipeptid-Additionsverbindungen und den daraus freigesetzten Dipeptiden.

In der Deutschen Offenlegungsschrift 2 801 238 ist die Herstellung einer Dipeptid-Aminosäureester-additionsverbindung aus Phenylalaninestern und einer Asparaginsäure, deren Aminogruppe durch einen Oxycarbonyl-, Benzyloxycarbonyl-, Benzoylrest bzw. einen aromatisch substituierten Sulfonyl-oder Sulfinylrest geschützt ist, unter Einwirkung einer Protease beschrieben. Nach der Zersetzung der Additionsverbindungen in wäßrig sauren Lösungen wird durch Hydrierung des Dipeptids die Aminoschutzgruppe abgetrennt. Auf diesem Wege ist der Süßstoff Aspartam zugänglich.

Überraschend hat sich gezeigt, daß neben den in der Deutschen Offenlegungsschrift aufgelisteten Schutzgruppen auch Phenacetyl-, Phenoxyacetyl-und Phenoxypropionylreste als Aminoschutzgruppen für die Peptidverknüpfung durch Proteasen verwendet werden können. Die beanspruchte Verfahrensweise ist vorteilhaft, da die erfindungsgemäßen Schutzgruppen wesentlich preiswerter einführbar sind als die schon bekannten Gruppen.

Das erfindungsgemäße Verfahren eröffnet einen neuen Weg zu dem Süßstoff Aspartam. Es ist nämlich möglich, entsprechend dem Belgischen Patent Nr. 902 474, in einer nachfolgenden Reaktionsstufe die erfindungsgemäße Schutzgruppe im pH-Bereich von etwa 5,0-7,5 unter Einwirkung von Amidasen sehr einfach und schonend wieder abzuspalten.

Die Erfindung betrifft somit:

1. Ein Verfahren zur Herstellung der Additionsverbindung der allgemeinen Formel I,

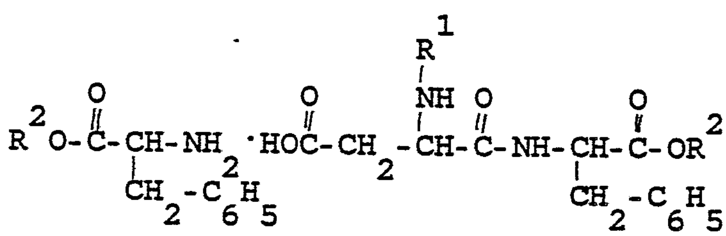

I

in der $R^1$ eine Aminoschutzgruppe und $R^2$ eine niedere Alkylgruppe ist, durch Verknüpfung einer N-geschützten L-Asparaginsäure mit einem D,L-Phenylalaninalkylester oder L-Phenylalaninalkylester in Gegenwart einer Protease, in einem pH-Bereich, in dem die Protease enzymatisch aktiv ist, das dadurch gekennzeichnet ist, daß ein Asparaginsäurederivat eingesetzt wird, dessen Aminogruppe durch eine Phenoxyacetyl-, Phenoxypropionyl-oder Phenylacetylgruppe geschützt ist.

2. Eine Additionsverbindung der allgemeinen Formel I, in der $R^1$ eine Phenoxyacetyl-, Phenoxypropionyl-oder Phenylacetylgruppe ist und $R^2$ die oben genannte Bedeutung hat.

3. Ein Verfahren zur Herstellung der Verbindung der allgemeinen Formel II,

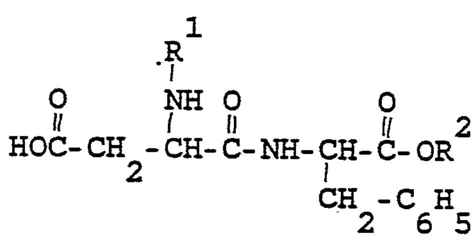

II

in der $R^1$ eine Aminoschutzgruppe und $R^2$ eine niedere Alkylgruppe ist, durch Zersetzung der Additionsverbindung der allgemeinen Formel I,

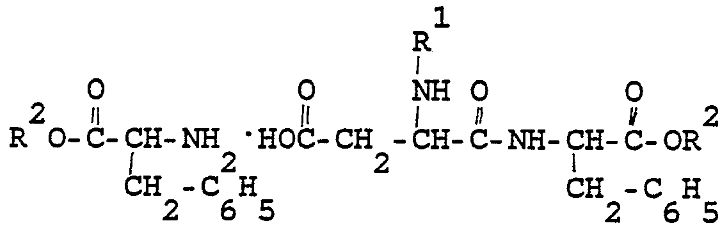

I

in der $R^1$ und $R^2$ die oben genannte Bedeutung haben, in wäßrig-saurer Lösung, das dadurch gekennzeichnet ist, daß man die Additionsverbindung der allgemeinen Formel I einsetzt, in der $R^1$ eine Phenoxyacetyl-, Phenoxypropionyl-oder Phenylacetylgruppe ist und $R^2$ die oben genannte Bedeutung hat.

In der folgenden Beschreibung wird die Erfindung genauer erläutert sowie in den Ansprüchen definiert.

In den allgemeinen Formeln I und II bedeutet $R^1$ eine Phenoxyacetyl-, Phenoxypropionyl-oder Phenylacetylgruppe und wird als Aminoschutzgruppe eingesetzt. $R^2$ ist eine niedere Alkylgruppe mit vorzugsweise 1-5 Kohlenstoffatomen, insbesondere 1-3 C-Atomen.

Die Herstellung der Additionsverbindung der allgemeinen Formel I und die Freisetzung der Verbindung der allgemeinen Formel II aus dieser Additionsverbindung erfolgt analog der Deutschen Offenlegungsschrift 2 801 238.

Durch enzymatische Reaktion entsteht aus einem Mol N-geschützter Asparaginsäure und einem Mol Phenylalaninester das entsprechende Dipeptid, das mit überschüssigem Phenylalaninester ein - schwerlösliches Addukt (Formel I) bildet und ausfällt. Es ist deshalb vorteilhaft, auf ein Mol N-geschützter Asparaginsäure 2-4 Mol Phenylalaninester einzusetzen. Da das Enzym nur die L-Aminosäuren verknüpft, ist es besonders vorteilhaft für ein Mol N-geschützter L-Asparaginsäure mindestens 2 Mol racemischen Phenylalaninester einzusetzen. Es entsteht dann das L,L-Dipeptid, während der D-Phenylalaninester im Addukt salzartig gebunden ist. Diese Verfahrensweise ermöglicht den Einsatz des preiswerteren racemischen Phenylalaninesters und die Gewinnung des enantiomerenreinen D-Phenylalaninesters aus dem Reaktionsgemisch als Nebenprodukt.

Als Phenylalaninester sind vorzugsweise solche Verbindungen geeignet, deren Alkoxyrest 1-5 Kohlenstoffatome enthält, also beispielsweise die Methyl-Ethyl-n-Propyl, i-Propyl-, n-Butyl, i-Butyl-, tert. Butyl-, n-Pentyl-, Neo-Pentylester, wobei die genannten $C_1$ bis $C_3$ -Alkylgruppen besonders bevorzugt sind. Auch höhere Alkoxyreste kommen noch in Frage, es wird aber immer schwieriger diese z.B. in wäßrig alkoholischem Medium zu lösen. Die Phenylalaninester können in freier Form oder in Form ihrer Salze, beispielsweise als Hydrochloride, Sulfate oder Acetate eingesetzt werden.

Die erfindungsgemäß eingesetzte N-geschützte Asparaginsäure ist durch an sich bekannte Verfahren leicht zugänglich. Vorteilhaft schützt man für die Herstellung des L,L-Dipeptides die Aminogruppe der verhältnismäßig preiswerten Asparaginsäure. Die N-geschützte Asparaginsäure kann in freier Form oder als Salz, beispielsweise als Na-, K-oder $NH_4$ -Salz, eingesetzt werden.

Um die Reaktion durchzuführen, ist die Einwirkung einer Protease erforderlich. Geeignet sind Enzyme, die Peptidbindungen spalten bzw. knüpfen, wie beispielsweise Bromelain, Ficin, Papain, Pepsin, Peptidase oder Trypsin. Besonders glatt und aufgrund der Thermostabilität auch bei relativ hohen Temperaturen verläuft die Verknüpfung bei Verwendung von Thermolysin oder Thermoase. Man verwendet pro Mol N-geschützter Asparaginsäure 1 g bis 100 g Enzym, vorzugsweise 5 g bis 50 g. Zu hohe Enzymzusätze sind unvorteilhaft wegen des hohen Preises und der schwierigen Aufarbeitung der Ansätze zur Rückgewinnung des Enzyms. Bei zu geringen Enzymzusätzen verläuft die Reaktion langsamer.

Das Enzym kann in Substanz, gelöst oder ungelöst, sowie in fixierter Form auf einem Träger eingesetzt werden. Zur dauernden Aktivierung des Enzyms sind Zusätze geringer Mengen von Calcium-und Zinkionen vorteilhaft. Die Reaktion wird in Lösungs-oder Verdünnungsmitteln durchgeführt. Besonders vorteilhaft sind solche Systeme, in denen die Ausgangsmaterialien gut löslich sind, während das gebildete Dipeptid in Substanz als Addukt oder Salz ausfällt. Da die Verknüpfung eine Gleichgewichtsreaktion ist, werden auf diese Weise besonders hohe Ausbeuten erhalten. Besonders geeignet sind Wasser und wäßrige Mischungen mit organischen Lösungsmitteln. Als solche sind geeignet: Alkohole, wie Methanol, Ethanol,i-Propanol, Butanol, Benzylalkohol, Ethylenglykol, Ethylenglykol-mono-methylether, Diglykol und Glycerin; organische Ester, wie Methylacetat, Ethylacetat, Butylacetat, Ethylenglykolmonoacetat, Ethylenglykol-diacetat und Diethylcarbonat; Ether, wie Diethylether, Di-i-Propylether, Ethylenglykol-dimethylether; Halogenkohlenwasserstoffe, wie Methylenchlorid, bzw. Säureamide, wie Dimethylformamid und Tetramethylharnstoff.

Bei der Verwendung von Lösungsmittelsystemen mit Mischungslücken kann beispielsweise in einem organischen Lösungsmittel gearbeitet werden, in dem sich eine emulgierte wäßrige Phase beispielsweise in Form des feuchten, fixierten Enzyms (Biotechnologie 3, Mai 1985, S. 459) oder in Form inverser Mizellen befindet [Ang. Chemie 97, 449 (1985)].

Die Reaktion ist wenig pH-abhängig. Sie wird vorteilhaft im pH-Bereich von 5-8 durchgeführt. Bei niedrigerem pH-Wert wird die N-geschützte Asparaginsäure immer schwerer löslich während bei höheren pH-Werten durch die Verseifung des Phenylalaninesters zunehmend Ausbeuteverluste entstehen. Die Reaktion wird im Temperaturbereich von etwa 20-50 °C durchgeführt. Bei tieferen Temperaturen verläuft sie langsamer, bei höheren Temperaturen verlieren die Enzyme allmählich ihre Aktivität.

Die Reaktanden können in beliebiger Reihenfolge auf einmal oder über eine längere Zeitspanne zudosiert werden.

Man kann beispielsweise das Enzym in wäßriger Lösung vorlegen und den Phenylalaninester sowie die N-geschützte Asparaginsäure in fester oder gelöster Form, als Salze oder in freier Form, getrennt oder in gemeinsamer Lösung zugeben. Man kann den Phenylalaninester mit dem Enzym vorlegen und die N-geschützte Asparaginsäure zudosieren. Man kann die N-geschützte Asparaginsäure mit dem Enzym vorlegen und den Phenylalaninester hinzufügen. Man kann auch die Lösung von N-geschützter Asparaginsäure und Phenylalaninester dem Enzym zusetzen.

Zur Gewinnung des Dipeptids wird das Reaktionsgemisch angesäuert, wobei der in der Additionsverbindung gebundene überschüßige Phenylalaninester in Lösung geht, während das freigesetzte Dipeptid abgesaugt oder mit einem Lösungsmittel extrahiert wird. Man kann auch die aus dem Reaktionsgemisch ausgefallene Additionsverbindung absaugen und das Fällungsprodukt in gleicher Weise in wäßrigen oder nicht-wäßrigen Phasen oder in Lösungsmittelgemischen mit Säure behandeln. Geeignete Säuren sind beispielsweise Salzsäure, Schwefelsäure, Essigsäure, die man vorteilhaft in verdünnter Form anwendet.

Beispiel 1:

Zu einer mit konz. Ammoniak auf pH 6 gestellten Lösung von 12,5 g (0,05 Mol) L-Phenylacetylasparaginsäure in 50 ml Wasser tropft man innerhalb von 16 Stunden bei 50 °C gleichzeitig eine Lösung von 21,5 g (0,1 M) D,L-Phenylalaninmethylester-hydrochlorid, 0,1 ml 4 %-ige Calciumchloridlösung, 0,1 ml 1 %-ige Zinkchloridlösung und 5,0 g Thermoase in 50 ml Wasser einerseits und konz. Ammoniak andererseits, so daß der pH-Wert von 6,0 aufrechterhalten bleibt. Man rührt 48 Stunden nach und saugt ab.

Man erhält 28,2 g (95 % d.Th.) Phenacetyl-L-Asp-L-Phe$OCH_3$. D-Phe$OCH_3$.

HPLC des Addukts zeigt die Zusammensetzung aus äquimolaren Mengen Phenylacetylaspartam und Phenylalaninmethylester: Säule RP8; Hypersil 5 µm; 250 × 4,6 nm; Flow: 1,0 ml/Min.

Phenylacetylaspartam: RT 16,35 Min.

L-Phenylalaninmethylester: RT 3,89 Min.

Beispiel 2:

Das gemäß Beispiel 1 erhaltene Produkt wird bereits in feuchtem Zustand in die eiskalte Mischung von 100 ml Schwefelsäure eingetragen und mit Ethylacetat extrahiert. Die organische Phase wird über $Na_2SO_4$ getrocknet und einrotiert.

Man erhält 19,3 g (94 % d.Th.) Phenacetyl-Aspartam.

$^1$H-NMR(DMSO); $\delta$ = 2,9 (m); 3,4 (s); 3,6 (s); 4,25-4,75 (m) 7,25 (m); 8,15-8,4 (q).

Bei Einstellung des pH-Wertes mit 33 %-iger Natron-oder Kalilauge statt konz. Ammoniak erhält man das Produkt in etwa gleicher Ausbeute.

Beispiel 3:

In die mit konz. Ammoniak auf pH 6 gestellte Lösung von 12,5 g (0,05 Mol) Phenylacetylasparaginsäure, 0,1 ml 4 %-ige Calciumchloridlösung, 0,4 ml 1 %-ige Zinkchloridlösung, 1,0 g in 35 %-iger NaBr-Lösung vorgelöstem Thermolysin trägt man bei 50° innerhalb von 3 Stunden 36,5 g (0,15 Mol) D,L-Phenylalanin-n-propylesterhydrochlorid unter gleichzeitiger Zugabe von konz. Ammoniak bei pH 6 ein. Nach 2 Stunden beginnt die klare Lösung zu kristallisieren. Nach 48 Stunden saugt man ab, verrührt unter gleichzeitiger Ultraschalleinwirkung mit 200 ml 2n Schwefelsäure und saugt ab.

Man erhält 21,1 g (96 % d.Th.) Phenacetyl-L-asparagyl-L-phenylalanin-propylester.
$^1$H-NMR (DMSO); $\delta$ = 0,8 (t); 1,45 (m) 2,95 (m); 3,4 (s); 3,9 (t); 4,3-4,7 (m) 7,25 (m) 8,2-8,45 (q).

## Ansprüche

1. Additionsverbindung der allgemeinen Formel I,

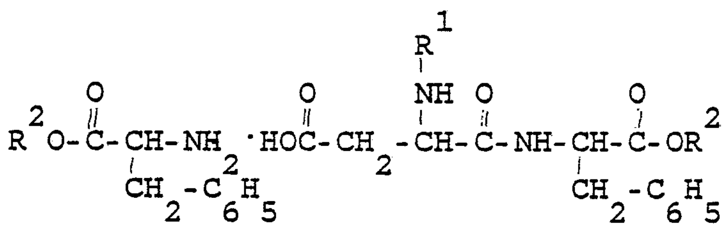

I

in der $R^1$ eine Phenoxyacetyl-, Phenoxypropionyl-oder Phenylacetylgruppe ist und $R^2$ eine niedere Alkylgruppe bedeutet.

2. Verbindung nach Anspruch 1, dadurch gekennzeichnet, daß $R^2$ eine Alkylgruppe mit 1 bis 5 Kohlenstoffatomen bedeutet.

3. Verbindung nach Anspruch 2, dadurch gekennzeichnet, daß $R^2$ Methyl, Ethyl, n-Propyl oder i-Propyl bedeutet.

4. Verfahren zur Herstellung der Additionsverbindung der allgemeinen Formel I,

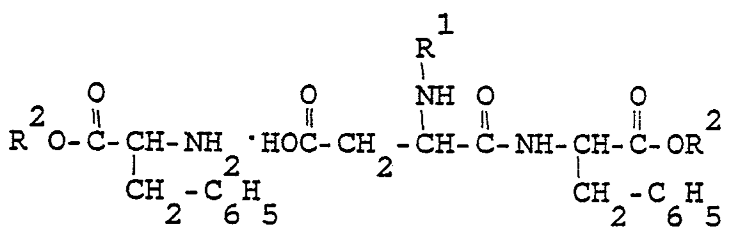

I

in der $R^1$ eine Aminoschutzgruppe und $R^2$ eine niedere Alkylgruppe sind, durch Verknüpfung einer N-geschützten L-Asparaginsäure mit einem D,L-Phenylalaninalkylester oder L-Phenylalaninalkylester in Gegenwart einer Protease, in einem pH-Bereich, in dem die Protease enzymatisch aktiv ist, dadurch gekennzeichnet, daß man eine Asparaginsäure einsetzt, in der $R^1$ eine Phenoxyacetyl-, Phenoxypropionyl- oder Phenylacetylgruppe bedeutet.

5. Verfahren nach Anspruch 4, dadurch gekennzeichnet, daß man einen D,L-Phenylalaninalkylester oder L-Phenylalaninalkylester einsetzt, in dem $R^2$ eine Alkylgruppe mit 1 bis 5 Kohlenstoffatomen bedeutet.

6. Verfahren nach Anspruch 5, dadurch gekennzeichnet, daß man einen Phenylalaninalkylester einsetzt, in dem $R^2$ Methyl, Ethyl, n-Propyl oder i-Propyl bedeutet.

7. Verfahren zur Herstellung der Verbindung der allgemeinen Formel II,

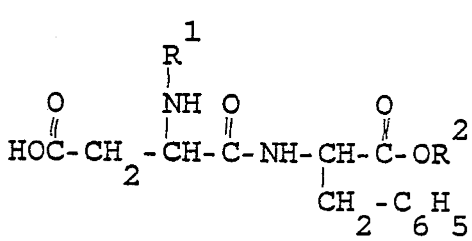

II

in der $R^1$ eine Aminoschutzgruppe und $R^2$ eine Alkylgruppe ist, durch Zersetzung der Additionsverbindung der allgemeinen Formel I,

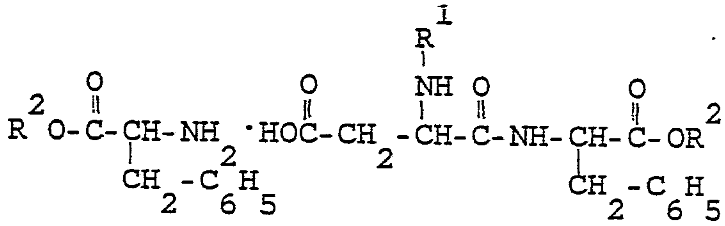

I

in der R¹ und R² die oben genannte Bedeutung haben, wäßrig-saurer Lösung, dadurch gekennzeichnet, daß man die Additionsverbindung der allgemeinen Formel I einsetzt, in der R¹ eine Phenoxyacetyl-, Phenoxypropionyl-oder Phenylacetylgruppe ist.

8. Verfahren nach Anspruch 7, dadurch gekennzeichnet, daß man die Additionsverbindung einsetzt, in der R² eine Alkylgruppe mit 1 bis 5 Kohlenstoffatomen bedeutet.

Patentansprüche für den folgenden Vertragsstaat: AT

1. Verfahren zur Herstellung der Additionsverbindung der allgemeinen Formel I.

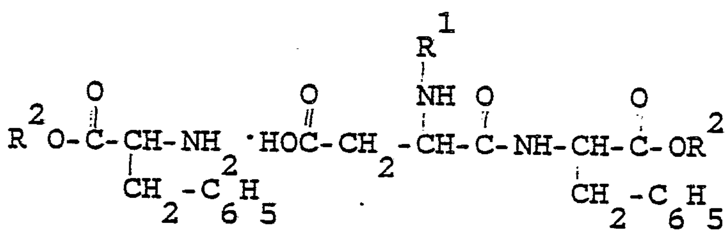

I

in der R¹ eine Aminoschutzgruppe und R² eine niedere Alkylgruppe sind, durch Verknüpfung einer N-geschützten L-Asparaginsäure mit einem D,L-Phenylalaninalkylester oder L-Phenylalaninalkylester in Gegenwart einer Protease, in einem pH-Bereich, in dem die Protease enzymatisch aktiv ist, dadurch gekennzeichnet, daß man eine Asparaginsäure einsetzt, in der R¹ eine Phenoxyacetyl-, Phenoxypropionyl- oder Phenylacetylgruppe bedeutet.

2. Verfahren nach Anspruch 1, dadurch gekennzeichnet, daß man einen D,L-Phenylalaninalkylester oder L-Phenylalaninalkylester einsetzt, in dem R² eine Alkylgruppe mit 1 bis 5 Kohlenstoffatomen bedeutet.

3. Verfahren nach Anspruch 2, dadurch gekennzeichnet, daß man einen Phenylalaninalkylester einsetzt, in dem R² Methyl, Ethyl, n-Propyl oder i-Propyl bedeutet.

4. Verfahren zur Herstellung der Verbindung der allgemeinen Formel II,

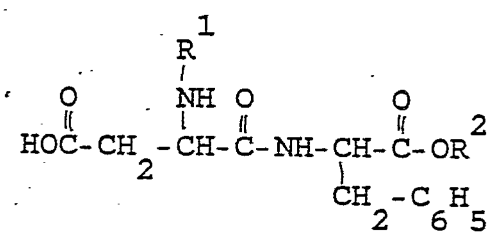

II

in der R¹ eine Aminoschutzgruppe und R² eine Alkylgruppe ist, durch Zersetzung der Additionsverbindung der allgemeinen Formel I,

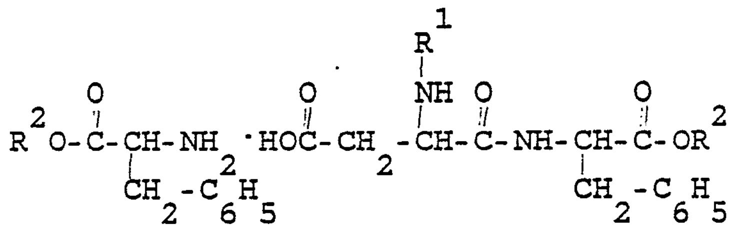

I

in der $R^1$ und $R^2$ die oben genannte Bedeutung haben, in wäßrig-saurer Lösung, dadurch gekennzeichnet, daß man die Additionsverbindung der allgemeinen Formel I einsetzt, in der $R^1$ eine Phenoxyacetyl-, Phenoxypropionyl-oder Phenylacetylgruppe ist.

5. Verfahren nach Anspruch 4, dadurch gekennzeichnet, daß man die Additionsverbindung einsetzt, in der $R^2$ eine Alkylgruppe mit 1 bis 5 Kohlenstoffatomen bedeutet.